# EUROPEAN PATENT APPLICATION

(11) **EP 1 939 163 A1**
(43) Date of publication of application: **02.07.2008**
(21) Application number: 06811837.1
(22) Date of filing: 16.10.2006
(51) Int. Cl.: C07C 37/20, B01J 8/02, C07C 39/16, C07B 61/00

(54) **FIXED-BED REACTOR AND PROCESS FOR PRODUCING 2,2-BIS(4-HYDROXYPHENYL)PROPANE WITH THE SAME**

(30) Priority: 20.10.2005 JP 2005305716
(71) Applicant: Idemitsu Kosan Co., Ltd., Chiyoda-ku Tokyo 100-8321 (JP)
(72) Inventor: YOSHITOMI, Kazuyuki, Chiba 299-0193 (JP); KODAMA, Masahiro, Chiba 299-0193 (JP); MASUDA, Shuichi, Chiba 299-0193 (JP); KOHIRUIMAKI, Jun, Chiba 299-0193 (JP); YAMASAKI, Hokuto, Chiba 299-0193 (JP)
(74) Representative: Vossius & Partner
(86) International application number: PCT/JP2006/320580
(87) International publication number: WO 2007/046338

(57) **Abstract**

A fixed-bed reactor has an ion-exchange resin catalyst layer. The reactor has a liquid collection/discharge mechanism in a lower part thereof, and a liquid reaction mixture yielded is discharged through the liquid collection/discharge mechanism. In the fixed-bed reactor, the liquid collection/discharge mechanism comprises: inflow pipes which have, at the front ends thereof, a screen or perforated plate for enabling the liquid reaction mixture to pass therethrough and which have lengths regulated so that the ends thereof conform to the curved bottom surface of the fixed-bed reactor; liquid collection pipes which have been connected to the back ends of the inflow pipes and serve to collect the liquid reaction mixture which has passed through the inflow pipes; and a liquid discharge pipe which has been connected to the liquid collection pipes and serves to discharge the liquid reaction mixture outside the system. Due to the constitution, the liquid reaction mixture passing through the packed-catalyst layer flows uniformly, and the lower part of the reactor can be prevented from having a space not filled with the catalyst. Thus, the fixed-bed reactor has an improved product yield per unit reactor volume per unit time. Also provided is a process for producing bisphenol A in which the reactor is used.

## Description

### Technical Field

The present invention relates to a fixed-bed reactor having a layer of ion-exchange resin catalyst and a process for producing 2,2-bis(4-hydroxyphenyl) propane (hereinafter, referred to bisphenol A) using the fixed-bed reactor.

### Background Art

In recent years, a technology of using ion-exchange resin layer as a catalyst for organic synthesis has been intensively studied, and various reactions using ion-exchange resins have been industrially practiced. This is because, as opposed to a situation where post-treatment such as catalyst removal from a reaction mixture is cumbersome and complicated when a conventional chemical is used as a catalyst, the use of ion-exchange resins as a catalyst has an advantage of easy post-treatment of the reaction mixture. In particular, a fixed-bed continuous reaction process, where granular ion-exchange resins are filled in a reactor and raw materials are continuously fed into the reactor, is advantageous because the post-treatment is extremely simple. As an example of using ion-exchange resins as a catalyst, there may be mentioned bisphenol A production. Bisphenol A is known as an important compound as a raw material for engineering plastics such as polycarbonate and polyallylate, epoxy resin, and the like, and the demand thereof tends to grow in recent years more and more. Bisphenol A is synthesized generally from acetone and phenol at 50°C to 90°C in the presence of a sulfonic acid cation-exchange resin catalyst. Conventionally, the sulfonic acid cation-exchange resin is filled in a reactor in a manner such that a liquid distributor and a screen, or a perforated plate are disposed at an upper and lower part inside of a reactor, and a catalyst is filled therebetween as shown in FIG. 3 or FIG. 4 (for example, see Patent Document 1).

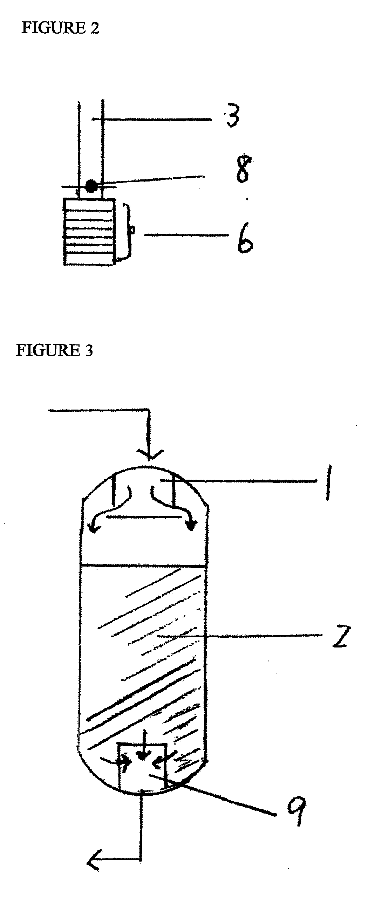

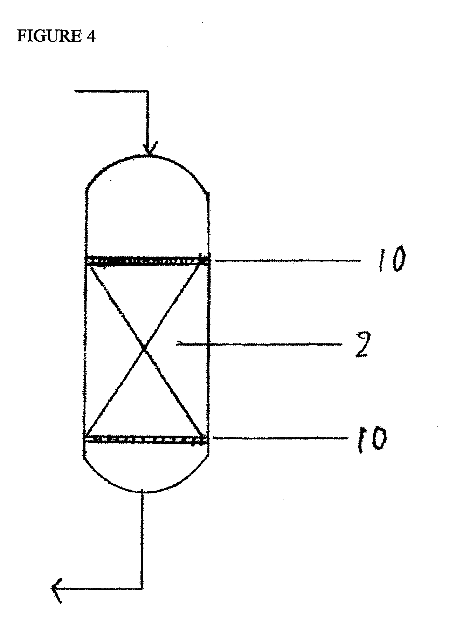

However, as shown in FIG. 3 when a liquid distributor 1 is disposed at the upper part of a reactor and a screen 9 is disposed at the lower part, the flow of a reaction liquid passing through a packed catalyst layer tends to become non-uniform and the discharging of a product liquid from the lower part also tends to become non-uniform. As a result, the problem is that the whole of the catalyst provided to the reaction is not fully utilized. Further, as shown in FIG. 4, when perforated plates 10 are used, the flow of the reaction liquid becomes uniform, but a space, so called a dead space where no catalyst is filled is formed in the lower part of the reactor, thereby decreasing the yield of bisphenol A per unit reactor volume per unit time.

Patent Document 1: Japanese Patent Application Laid-Open No. H06-340563

### Disclosure of Invention

### Problems to be Solved by the Invention

The present invention has been made under these circumstances. An object of the present invention is to provide a fixed-bed reactor having a layer of an ion-exchange resin catalyst and a method for producing bisphenol A using the reactor. The fixed-bed reactor makes uniform the flow of a reaction liquid passing through the packed catalyst layer, eliminating a space not filled with the catalyst in the lower part of the reactor, improving the yield of a product per unit reactor volume per unit time.

### Means for Solving the Problems

The present inventors have made intensive studies to achieve the above object. As a result, the inventors have found that, by providing a liquid collecting/discharging mechanism with an unconventional structure that collects/discharges a product liquid at the bottom of a fixed-bed reactor, the fixed-bed reactor has been made to flow a reaction liquid passing through a catalyst layer uniformly, to eliminate a space not filled with a catalyst in the lower part of the reactor, and to improve the yield of a product per unit reactor volume per unit time. Based on these findings, the present invention has been accomplished.

The present invention provides:
(1) A fixed-bed reactor having an ion-exchange resin catalyst layer and provided, in a lower part thereof, with a liquid collecting/discharging mechanism through which a reaction product liquid is discharged, the liquid collecting/discharging mechanism comprising:
   a plurality of inflow pipes provided, at a front end thereof, with a screen or perforated plate for flowing in the reaction product liquid and having a length conforming to a curved surface at the bottom of the fixed-bed reactor;
   a plurality of liquid collecting pipes connected to a back end of the inflow pipes for collecting the reaction product liquid that has passed through each of the inflow pipes; and
   a liquid discharging pipe connected to the liquid collecting pipes for discharging the reaction product liquid outside the system.
(2) The fixed-bed reactor as described in (1), wherein an opening of the screen or the perforated plate is 0.4 mm or less;
(3) The fixed-bed reactor as described in (1), wherein an opening of the screen or the perforated plate is 0.2 mm or less;
(4) The fixed-bed reactor as described in any one of (1) to (3), wherein an orifice is inserted in a front end of the inflow pipe;
(5) The fixed-bed reactor as described in any one of (1) to (4), wherein 5 or more inflow pipes are disposed per unit cross-sectional area (m²) of the reactor;
(6) The fixed-bed reactor as described in any one of (1) to (4), wherein 8 or more inflow pipes are disposed per unit cross-sectional area (m²) of the reactor;
(7) The fixed-bed reactor as described in (1), wherein the distance between the screen or perforated plate and a curved surface at the bottom of the fixed-bed reactor is 300 mm or less;
(8) The fixed-bed reactor as described in (1), wherein the distance between the screen or the perforated plate and a curved surface at the bottom of the fixed-bed reactor is 100 mm or less;
(9) The fixed-bed reactor as described in any one of (1) to (8), used for the production of 2,2-bis(4-hydroxyphenyl) propane; and
(10) A process for producing 2,2-bis(4-hydroxyphenyl) propane comprising using a fixed-bed reactor as described in (9).

### Effect of the Invention

The present invention provides a fixed-bed reactor that makes the flow of a reaction liquid passing through a packed catalyst layer uniform, eliminates a space not filled with a catalyst in the lower part of the reactor, and improves the yield of a product per unit reactor volume per unit time. The present invention also provides a process for producing bisphenol A using the reactor.

### Brief Description of the Drawings

FIGURE 1 illustrates an example of a fixed-bed reactor of the present invention having a liquid collecting/discharging mechanism in a lower part thereof; (a) is a schematic side view and (b) is a schematic plan view of the liquid collecting/discharging mechanism.
FIGURE 2 illustrates an example of the structure of a front end of an inflow pipe.
FIGURE 3 illustrates an example of a conventional fixed-bed reactor having a liquid distributor.
FIGURE 4 illustrates an example of a conventional fixed-bed reactor having a perforated plate.

### Reference Marks in the Drawings

1: Liquid distributor in an upper part of a reactor
2: Layer packed with ion-exchange resin catalyst
3: Inflow pipe
4: Liquid collecting pipe
5: Liquid discharging pipe
6: Screen or perforated plate
7: Liquid collecting/discharging mechanism
8: Orifice
9: Screen
10: Perforated plate

### Best Mode for Carrying Out the Invention

A fixed-bed reactor of the present invention is a reactor having a layer of an ion-exchange resin catalyst. The reactor has a liquid collecting/discharging mechanism in a lower part thereof. A reaction product liquid is discharged through the liquid collecting/discharging mechanism. The liquid collecting/discharging mechanism is characterized by having: plural inflow pipes designed in such a way that a screen or perforated plate allowing the reaction product liquid to flow in is supplied at a front end thereof and that a length of the pipe conforms to a curved surface at the bottom of the fixed-bed reactor; plural liquid collecting pipes that are connected to the back ends of the inflow pipes and serve to collect the reaction product liquid passing through each of the inflow pipes; and a liquid discharging pipe that is connected to the liquid collecting pipes and serves to discharge the reaction product liquid outside the system.

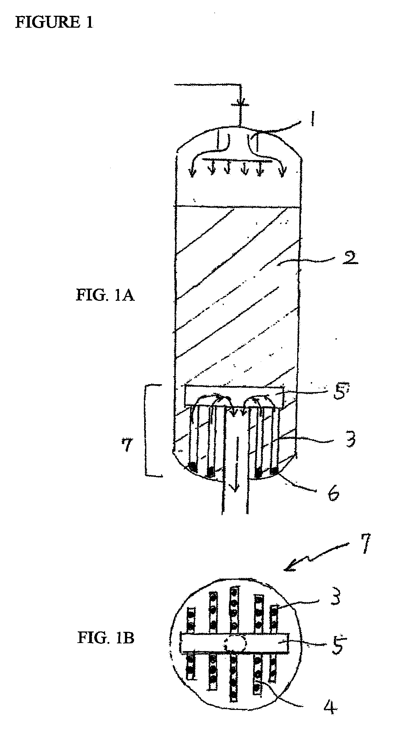

FIGURE 1 illustrates an example of a fixed-bed reactor of the present invention having a liquid collecting/discharging mechanism in the lower part thereof.
FIGURE 1a is a schematic side view.
FIGURE 1b is a schematic plan view of the liquid collecting/discharging mechanism. The liquid collecting/discharging mechanism is referenced by the number of "7".
A raw material liquid passes through a liquid distributor 1 disposed in an upper part of the fixed-bed reactor and brought into contact with a layer of an ion-exchange resin catalyst 2, so that reaction proceeds. Reaction product liquid flows into plural inflow pipes 3 through a screen or perforated plate 6 disposed at a front end of the inflow pipe. The inflow pipe 3 is designed in such a way that a length of the pipe conforms to a curved surface at the bottom of the fixed-bed reactor.
The distance between the screen or the perforated plate and the curved surface at the bottom of the fixed-bed reactor is preferably 300 mm or less and more preferably 100 mm or less in order to keep the uniformity of the reaction product liquid flow.
After passing through each inflow pipe 3, the product liquid is collected by plural liquid collecting pipes 4 that are connected to a back end of the inflow pipes 3, then the reaction product liquid is discharged outside the reactor system through a liquid discharging pipe 5 that is connected to each of the liquid collecting pipes 4.
The inflow pipe 3 is preferably disposed nearly in parallel to the reactor wall. The liquid collecting pipe 4 that is connected to the back end of the inflow pipe 3 is preferably disposed nearly perpendicular to the reactor wall. Further, the liquid collecting pipe 4 is preferably disposed at a position away from the lowermost part of the reactor by a distance of about 3/20 to about 8/20 of the length of the layer of an ion-exchange resin catalyst.

As opposed to the fixed-bed reactor shown in FIG. 4 having a perforated plate in a lower part of the reactor, the liquid collecting/discharging mechanism 7 equipped in accordance with the present invention allows the lower part of the reactor to be free from a space, what is called a dead space where no catalyst is filled and a reaction liquid injected from the upper part of the reactor to flow down uniformly through the packed catalyst layer, so that a uniform reaction proceeds, thereby fully utilizing the whole of the catalyst provided to the reaction, letting a reaction product liquid that flows down be withdrawn out of the reactor through the liquid collecting/discharging mechanism without staying at the bottom of the reactor, and improving the yield of a product per unit reactor volume per unit time. Further, in order to prevent deterioration of the quality of the obtained product, it is quite important that, after the ion-exchange resin catalyst is filled in the reactor, the sulfonic acid remaining in the ion-exchange resin catalyst be fully removed by water washing before the reaction. In this case, the liquid collecting/discharging mechanism thus equipped allows the packed ion-exchange resin catalyst layer to be water-washed uniformly, thereby eliminating the sulfonic acid remaining due to partly insufficient water-washing. Further, the inside of the reactor is water-washed when the spent catalyst is withdrawn out of the reactor. In this case, another advantage is that water washing is easily carried out because there is no portion disturbing the flow of the washing water in the reactor.
Still further, there is provided with a mechanism for discharging a liquid collected in the lower part of the reactor by installing a drain nozzle that has the same screen as the liquid collecting pipes 4 in the lowermost part of the reactor.

FIGURE 2 illustrates an example of the structure of the front end of the inflow pipe 3. As shown in FIG. 2, at the front end of the inflow pipe 3 is disposed a screen or perforated plate that allows a reaction liquid to flow into the inflow pipe 3. An opening of the screen or perforated plate 6 disposed at the front end of the inflow pipe 3 is not particularly limited as far as the ion-exchange resin catalyst utilized does not come into the inflow pipe 3, and the opening is preferably 0.4 mm or less and more preferably 0.2 mm or less. As such a screen, "Johnson screen" is mentioned.

In the present invention, an orifice 8 is preferably inserted in the front end of each inflow pipe 3 as shown in FIG. 2 so as to make a liquid flow more uniform. By inserting the orifice, the flow rate of the reaction product liquid per inflow pipe may be controlled, the product liquid is prevented from staying at the bottom of the reactor, the reaction proceeds uniformly in the catalyst-filled layer and the whole of the filled catalyst is fully utilized. As a result, the yield of a product per unit reactor volume per unit time can be increased.

In the present invention, the number of the inflow pipes 3 is not particularly limited, and is preferably 5 or more per unit cross-sectional area of the reactor (m²) and more preferably 8 or more. This allows the reaction liquid to flow down uniformly through the packed catalyst layer; the whole packed catalyst can be fully utilized, and the product liquid is prevented from staying at the bottom of the reactor. As a result, the yield of a product per unit reactor volume per unit time can be increased.

The process for producing bisphenol A of the present invention is characterized by using the fixed-bed reactor of the present invention described above.
In the process for producing bisphenol A, generally a continuous fixed-bed reaction process is used, where a raw material mixture liquid containing phenol and acetone is fed continuously to a reactor packed with a cation-exchange resin.
The acetone is mixed with a large excess of phenol by a line mixer and the like, adjusted to a predetermined temperature with a heat exchanger placed at the inlet of the fixed-bed reactor, and then fed as a down-flow stream into the fixed bed reactor.
In the case where two or more fixed-bed reactors are disposed in series, fresh acetone may be mixed with a large excess of phenol by a line mixer and the like, adjusted to a predetermined temperature with a heat-exchanger placed at the inlet of a first fixed-bed reactor, and then fed into the first fixed-bed reactor, or after acetone required for the reaction is separated into portions and fed into each fixed-bed reactor, each portion of the acetone may be mixed with a large excess of phenol by a line mixer, adjusted to a predetermined temperature with a heat-exchanger disposed at the inlet of each fixed-bed reactor, and then fed into each fixed-bed reactor.

Preferable as a coolant for the heat exchanger is a hot water having the same temperature as the solidifying temperature of the reaction liquid or a temperature slightly higher (for example, approximately 2°C higher than the solidifying temperature of the reaction liquid). With the hot water passing through, the reaction liquid can be prevented from being solidified in the heat exchanger.
The difference between the inlet temperature and outlet temperature of the fixed-bed reactor is preferably 25°C or less and more preferably 15°C or less. Within the above temperature difference, formation of impurities is suppressed and there is attained a good selectivity for bisphenol A.
The reaction pressure is selected as about 0.1 to about 1 MPa, and the pressure difference ΔP in the fixed bed reactor is about 0.05 to about 0.15 MPa, which are generally adopted as operating conditions for commercial reactors.
The ratio of tower height (L, straight part) to tower diameter (D) of the fixed-bed reactor, L/D is preferably about 0.5 to about 1.0 as a commercial reactor. Within this range, channeling of the reaction liquid does not occur in the reactor, and ΔP (pressure drop) problem does not arise, either.
Further, an electric heater or a hot water-flowing pipe trace or jacket is preferably installed outside the fixed-bed reactor so as to prevent solidification of the reaction liquid in the reactor when the reaction is terminated. In view of preventing deterioration of product quality caused by thermal degradation of the ion-exchange resin catalyst that is brought about by local elevation of temperature, keeping the temperature with hot water is preferable.

To the raw material mixture liquid, besides phenol and acetone, an auxiliary catalyst may be admixed optionally to enhance selectivity or reaction rate. The auxiliary catalyst includes, for example, alkyl mercaptans such as methyl mercaptan, ethyl mercaptan, and n-octyl mercaptan; thiocarboxylic acids such as thioglycolic acid and β-mercaptopropionic acid; aminoalkanethiols such as 2-aminoethanethiol; mercaptoalcohol such as mercaptoethanol; and the like. The molar ratio of acetone/phenol is selected in the range of generally from 1/30 to 1/3 and preferably from 1/15 to 1/5. Within the above range of the molar ratio, the reaction rate is not lowered, also formation of impurities is prevented, thereby preventing lowering of the selectivity of bisphenol A. The molar ratio of the auxiliary catalyst/acetone is selected in the range of generally 0.1/100 to 20/100 and preferably 1/100 to 10/100. Within the above range of the molar ratio, the effect of enhancing the reaction rate and selectivity of bisphenol A is sufficiently exhibited.
These auxiliary catalysts may be fixed on the acid ion-exchange resin mentioned above to serve as an auxiliary catalyst.

The ion-exchange resin catalyst used is not particularly limited, and ion-exchange resin catalysts usually used for bisphenol A production may be used. Preferably used as the foregoing ion-exchange resin catalysts, for example, is a sulfonic acid ion-exchange resin. Acceptable as the sulfonic acid ion-exchange resin is a strongly acidic cation-exchange resin having sulfonic acid groups, which includes sulfonated styrene/divinylbenzene copolymer, sulfonated cross-linked styrene polymer, phenol-formaldehyde-sulfonic acid resin, benzene-formaldehyde-sulfonic acid resin, and the like. These may be used singly in one kind or in a combination of two or more kinds.

The reaction temperature is selected in the range of generally 40 to 150°C and preferably 60 to 110°C. At a reaction temperature lower than 40°C, the reaction rate is low and the viscosity of the reaction liquid is extremely high, and possibly the reaction liquid is solidified in some cases. When the reaction temperature exceeds 150°C, the control of the reaction is difficult, the bisphenol A selectivity is lowered, and the cation-exchange resin serving as the catalyst is possibly decomposed or degraded. The liquid hourly space velocity (LHSV) is selected within the range of generally 0.2 to 30 hr⁻¹ and preferably from 0.5 to 6 hr⁻¹. In this way, the product liquid coming out of the reactor packed with the catalyst is subjected to a post-treatment in a conventional process and bisphenol A is taken out.
In an apparatus equipped with a fixed-bed reactor, filters are preferably disposed at predetermined positions so as to remove foreign matters such as disintegrated cation-exchange resin catalyst. The positions at which the filters are disposed include the outlet of the fixed-bed reactor or the crystallization step where a bisphenol A-phenol adduct is separated from the product liquid. The material of the filters used is preferably sintered metal, glass wool, polypropylene (PP), polyphenylene sulfide (PPS), and polyamide.

Next, an example of the post-treatment is explained. Firstly, concentration is performed before crystallization. The conditions for concentration are not particularly limited, and concentration is generally performed under the conditions including the temperature ranging from 130 to 170°C and the pressure ranging from 13.3 to 53.3 kPa. At a temperature lower than 130°C, a high vacuum is required. Temperature exceeding 170°C increases impurities or causes coloring. The bisphenol A concentration in the liquid remaining after the concentration is advantageously in the range of 25 to 40 % by mass. When the concentration is lower than 25 % by mass, the recovery percentage of bisphenol A is low. When the concentration exceeds 40 % by mass, slurry transportation after crystallization is difficult. Crystallization of the bisphenol A-phenol adduct from the liquid remaining after concentration is usually performed by cooling crystallization. The crystallization temperature is preferably from 40 to 70°C. When the crystallization temperature is within this range, there is no possibility of viscosity increase or solidification of the crystallization liquid, and there is no increase in the dissolution loss of bisphenol A either.
Secondly, the bisphenol A-phenol adduct crystallized in this way is separated in a conventional process, and is usually washed with phenol. Subsequently, the washed adduct is decomposed into bisphenol A and phenol at a temperature of generally 130 to 200°C and preferably 150 to 180°C and under a pressure of generally 2.6 to 20 kPa. By removing substantially all the phenol remaining in the bisphenol A obtained through the above decomposition process through a process such as steam stripping, bisphenol A of high quality is obtained.

### Example

The present invention will be further described in detail with reference to the following examples, but it should be noted that the present invention is in no way limited by those examples.

### Example 1

A fixed-bed reactor (screen opening: 0.2 mm, distance between the screen and a curved surface at the bottom of the reactor: 100 mm, and liquid collecting pipes/catalyst filled-layer height = 6/20) was fabricated. The fixed-bed reactor was composed of a reactor (1:2 semi-ellipsoid, cross-sectional area: 2.5 m², height: 1 m, and volume: 4.1 m³) that was equipped, at the lower part thereof, with a liquid collecting/discharging mechanism provided with 25 pieces of inflow pipe 3 that had a structure shown in FIG. 1. Filled in this reactor was 3.3 m³ of a cation-exchange resin ("Diaion SK-104H" (trade name) manufactured by Mitsubishi Chemical Corp.) having 20% of acid sites modified by 2,2-dimethylthiazolidine as a catalyst. A mixed liquid of acetone and phenol (acetone/phenol molar ratio = 1:30) adjusted at 70°C was fed in a downflow stream at 20 m³/hr into the reactor so as to obtain bisphenol A. The yield of bisphenol A was 6%. The yield of bisphenol A per unit reactor volume per unit time was 1.5%/(m³ ·hr).

### Comparative Example

A fixed-bed reactor was fabricated, which was composed of a reactor (1:2 semi-ellipsoid, cross-sectional area: 2.5 m², height: 1 m, and volume: 4.1 m³) having a liquid collecting/discharging mechanism composed of upper and lower perforated plates. A cation-exchange resin similar to the one in example 1 as a catalyst was filled in this reactor. The ion-exchange resin catalyst was filled only in an amount of 2.5 m³ between the upper and lower perforated plates. Then, the same reaction as in example 1 was carried out to obtain bisphenol A. The yield of bisphenol A was 4.5%. The yield of bisphenol A per unit reactor volume per unit time was 1.1%/(m³ hr).

### Industrial Applicability

The present invention provides a fixed-bed reactor that makes uniform the flow of a reaction liquid passing through the packed catalyst layer, eliminates a space not filled with a catalyst in the lower part of the reactor, and improves the yield of a product per unit reactor volume per unit time. The present invention also provides a method for producing bisphenol A using the reactor.

## Claims

1. A fixed-bed reactor having an ion-exchange resin catalyst layer and provided, in a lower part thereof, with a liquid collecting/discharging mechanism through which a reaction product liquid is discharged, the liquid collecting/discharging mechanism comprising:
a plurality of inflow pipes provided, at a front end thereof, with a screen or perforated plate for flowing in the reaction product liquid and having a length conforming to a curved surface at the bottom of the fixed-bed reactor;
a plurality of liquid collecting pipes connected to a back end of the inflow pipes for collecting the reaction product liquid that has passed through each of the inflow pipes; and
a liquid discharging pipe connected to the liquid collecting pipes for discharging the reaction product liquid outside the system.

2. The fixed-bed reactor according to claim 1, wherein an opening of the screen or the perforated plate is 0.4 mm or less.

3. The fixed-bed reactor according to claim1, wherein an opening of the screen or the perforated plate is 0.2 mm or less.

4. The fixed-bed reactor according to any of claims 1 to 3, wherein an orifice is inserted in a front end of the inflow pipe.

5. The fixed-bed reactor according to any of claims 1 to 4, wherein 5 or more inflow pipes are disposed per unit cross-sectional area (m²) of the reactor.

6. The fixed-bed reactor according to any of claims 1 to 4, wherein 8 or more inflow pipes are disposed per unit cross-sectional area (m²) of the reactor.

7. The fixed-bed reactor according to claim 1, wherein the distance between the screen or perforated plate and the curved surface at the bottom of the fixed-bed reactor is 300 mm or less.

8. The fixed-bed reactor according to claim 1, wherein the distance between the screen or perforated plate and the curved surface at the bottom of the fixed-bed reactor is 100 mm or less.

9. The fixed-bed reactor according to any of claims 1 to 8 used for the production of 2,2-bis(4-hydroxyphenyl) propane.

10. A process for producing 2,2-bis(4-hydroxyphenyl) propane comprising using the fixed-bed reactor according to claim 9.
